# EUROPEAN PATENT APPLICATION

(11) **EP 4 834 377 A1**
(43) Date of publication of application: **04.11.2026**
(21) Application number: 25183804.1
(22) Date of filing: 18.06.2025
(51) Int. Cl.: A61L 9/12, H05K 7/12

(54) **MAGNETIC FRAGRANCE CARD**

(30) Priority: 02.05.2025 TW 114204432 U
(71) Applicant: Blueprint Technology Co., Ltd., Taipei City 106 (TW)
(72) Inventor: WU, Hua Ting, Taipei City, Taiwan, R.O.C. (TW)
(74) Representative: Savi, Massimiliano

(57) **Abstract**

A magnetic fragrance card has a card base (10). A side surface of the card base (10) forms a first embedding unit (14), at least one second embedding unit (15), and a first placement slot (11). A first magnetic member (20) is mounted in the first placement slot (11). Multiple support units (40) are mounted on the side surface of the card base (10). A fragrance member (50) is sleeved on the first embedding unit (14) and the second embedding unit (15). An outer cover (60) is penetrated through by a cover hole (62). The outer cover (60) covers on the card base (10) and is combined with each one of the support units (40). A convex unit (80) is mounted through the cover hole (62) and is combined and fixed with the first embedding unit (14) and the second embedding unit (15).

## Description

The present invention relates to a fragrance device, especially to a magnetic fragrance card.

These days people have higher standards for the quality of life. Even living in an environment with poor air quality, people are more and more meticulous about the smell of the air, and a fragrance device has been developed. One kind of fragrance device is capable of being mounted on a back side of a mobile phone. The user drops essential oils into the fragrance device, thereby improving the smell of the surrounding environment through the fragrance emitted when using the mobile phone for a long time, thereby soothing emotions.

However, the aforementioned fragrance device is small in size and can only hold a limited amount of essential oil. Therefore, the fragrance cannot be maintained for a long time, and the user needs to refill the essential oil from time to time to maintain the fragrance, which is inconvenient to use. Moreover, the small size of the fragrance device can only emit fragrance within a very limited range. Therefore, it is necessary that the user considers how to prolong the time for the fragrance device to emit fragrance and how to increase the range of its fragrance diffusion.

To overcome the shortcomings, the present invention provides a magnetic fragrance card to mitigate or obviate the aforementioned problems.

The main objective of the present invention is to provide a magnetic fragrance card that accommodates a large fragrance member in the space combined with the card base and the outer cover, and the magnetic fragrance card forms side penetrating holes, so that the magnetic fragrance card is slim and light, and diffuses the fragrance evenly over a wide range for a long time.

The magnetic fragrance card has a card base, a first magnetic member, multiple support units, a fragrance member, an outer cover, and a convex unit.

A side surface of the card base forms a first embedding unit, at least one second embedding unit, and a first placement slot. The first embedding unit and the at least one second embedding unit protrude from the card base, and the first placement slot is located on the card base.

A first magnetic member is mounted in the first placement slot. Multiple support units are mounted on the side surface of the card base.

A fragrance member is penetrated through by a first embedding hole and at least one second embedding hole. The first embedding hole and the at least one second embedding hole respectively correspond in shape and position to the first embedding unit and the at least one second embedding unit. The fragrance member is sleeved on the first embedding unit by the first embedding hole, and the fragrance member is sleeved on the at least one second embedding unit by the at least one second embedding hole.

An outer cover is penetrated through by a cover hole, and the outer cover covers the card base and is combined with each one of the support units.

A first embedding convex portion and at least one second embedding convex portion protrude from a side surface of the convex unit. The convex unit is mounted through the cover hole and is combined and fixed with the first embedding unit by the first embedding convex portion, and the convex unit is combined and fixed with the second embedding unit by the at least one second embedding convex portion.

When the magnetic fragrance card is installed on an electronic device having a magnetic area, the card base is magnetically mounted on the magnetic area of the electronic device by the magnetic member. The back side of the magnetic fragrance card and the surface of the electronic device are magnetically connected to each other, and the card base and the electronic device can be quickly combined or separated and is easy to carry. Multiple side penetrating holes are formed among and enclosed by the outer cover, the support units and the card base, the fragrance member disposed inside can have a larger volume, and also the fragrance of the fragrance member can be emitted from each one of the side penetrating holes and the cover hole of the outer cover, so as to emit fragrance for a long time multi-directionally, enhancing the diffusion uniformity of the fragrance and the comfort of the user. The magnetic fragrance card comprises the convex unit, preventing the user or external objects from directly touching the fragrance member, thereby protecting the fragrance member from being touched.

Other objectives, advantages and novel features of the invention will become more apparent from the following detailed description when taken in conjunction with the accompanying drawings.

### In the drawings:

Fig. 1 is a perspective view of a magnetic fragrance card of the first embodiment in accordance with the present invention;
Fig. 2 is an exploded view of the magnetic fragrance card in Fig. 1 of the first embodiment;
Fig. 3 is another exploded view of the magnetic fragrance card in Fig. 1 of the first embodiment;
Fig. 4 is a partial exploded view of the magnetic fragrance card in Fig. 1 of the first embodiment;
Fig. 5 is a cross-sectional view of the magnetic fragrance card in Fig. 1 of the first embodiment;
Fig. 6 is a schematic view of the magnetic fragrance card in Fig. 1 of the first embodiment, shown in a state of use;
Fig. 7 is a combinational view of the magnetic fragrance card in Fig. 1 of another embodiment;
Fig. 8 is a perspective view of a magnetic fragrance card of the second embodiment in accordance with the present invention;
Fig. 9 is an exploded view of the magnetic fragrance card in Fig. 8 of the second embodiment;
Fig. 10 is another exploded view of the magnetic fragrance card in Fig. 8 of the second embodiment; and
Fig. 11 is an exploded view of the magnetic fragrance card in Fig. 8 of another embodiment.

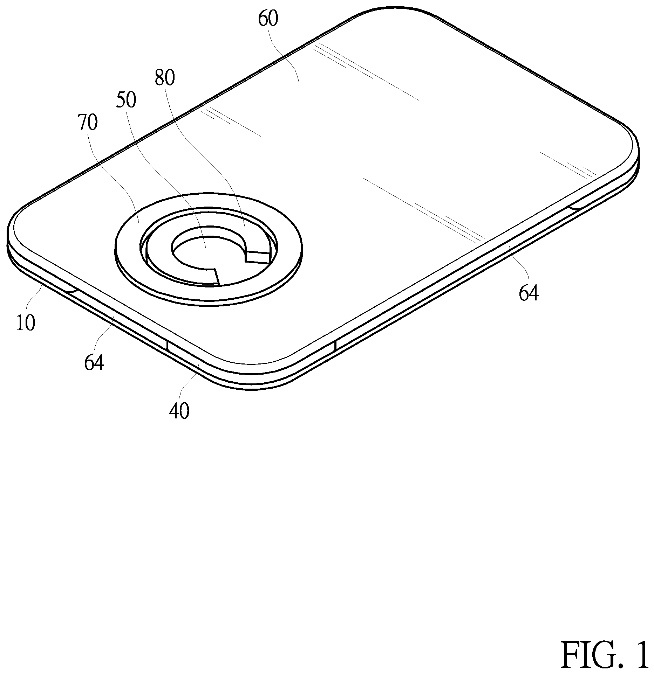

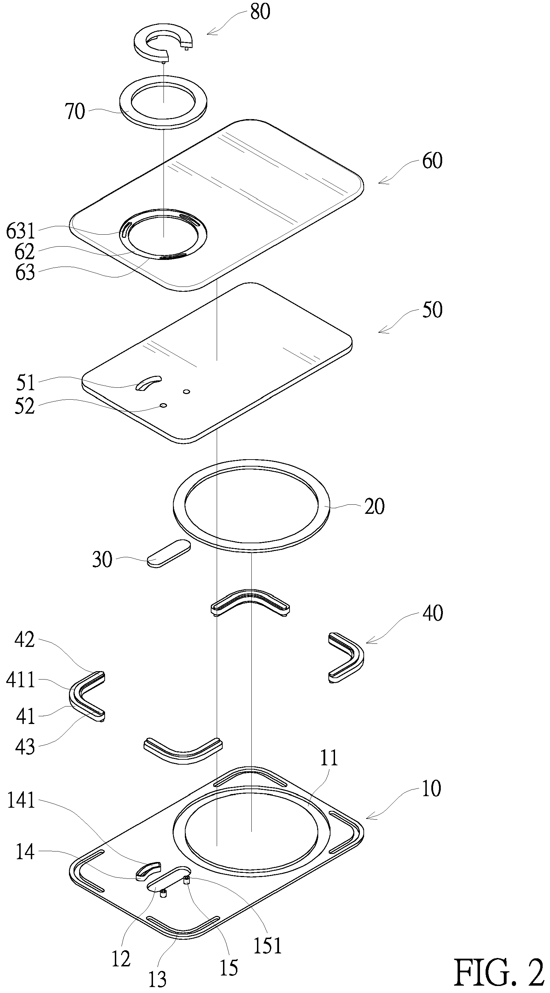

With reference to Fig. 1 and Fig. 2, a magnetic fragrance card in accordance with the present invention comprises a card base 10, a first magnetic member 20, a second magnetic member 30, multiple support units 40, a fragrance member 50, an outer cover 60, a decorative cover 70, and a convex unit 80. The first magnetic member 20 and the second magnetic member 30 are mounted on the card base 10. Each one of the support units 40 is mounted around an annular lateral edge of the card base 10. The fragrance member 50 is mounted on the card base 10 and located among the support units 40. The outer cover 60 covers the card base 10. The decorative cover 70 and the convex unit 80 are mounted through the card base 10.

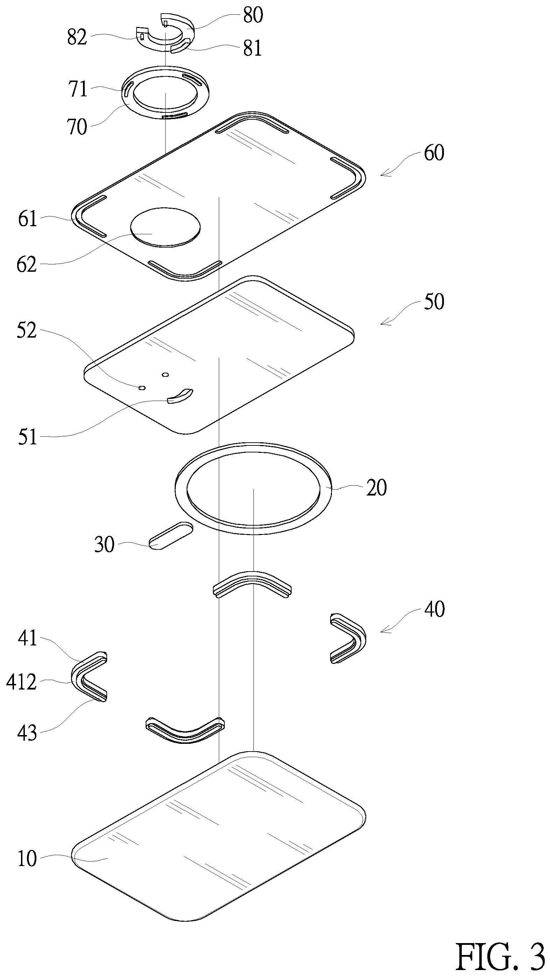

With reference to Fig. 2 and Fig. 3, the card base 10 is a rectangular plate, and the card base 10 comprises two opposite side edges on the left and right of the card base 10 and two opposite end edges on the front and rear of the card base 10. A side surface of the card base 10 is concave to form a first placement slot 11, a second placement slot 12, and multiple lower support slots 13. The first placement slot 11 is an annular groove and is located on an end of the card base 10. The second placement slot 12 is a straight groove and is located on the other end of the card base 10. Each one of the lower support slots 13 is an L-shaped groove and is respectively located on four corners of the card base 10. Each one of the lower support slots 13 extends along one of the side edges and one of the end edges adjacent to the said side edge, but it is not limited thereto. Whether the first placement slot 11, the second placement slot 12, and the lower support slot 13 are formed and the shapes of the formed slots can be changed according to the user. The card base 10 further forms a first embedding unit 14 and at least one second embedding unit 15. The first embedding unit 14 in the embodiment is an arc-shaped block protruding from the side surface of the card base 10 and located on a side of the second placement slot 12. A top surface of the first embedding unit 14 is concave to form a first embedding slot 141. The at least one second embedding unit 15 is a columnar structure. The quantity of the second embedding unit 15 is two, and the second embedding unit 15 protrudes from the other side of the second placement slot 12. A top surface of each one of the second embedding units 15 is concave to form a second embedding slot 151, but it is not limited thereto, as the shapes and the quantities of the first embedding unit 14 and the second embedding unit 15 can be changed according to the user.

The first magnetic member 20 is an annular magnet mounted in the first placement slot 11. The second magnetic member 30 is an elongated magnet and is mounted in the second placement slot 12, and the aforementioned components can be fixed by adhesive or heat welding, but it is not limited thereto. The configurations of the first magnetic member 20 and the second magnetic member 30, and the combination methods of the first magnetic member 20 and the second magnetic member 30 can be changed according to the user, and the second magnetic member 30 may be omitted and the second placement slot 12 may be omitted.

Each one of the support units 40 comprises a main body 41, an upper convex portion 42, and a lower convex portion 43. The main body 41 is elongated and comprises an upper surface 411 and a lower surface 412. The upper convex portion 42 protrudes from the upper surface 411. The lower convex portion 43 protrudes from the lower surface 412 and forms an elongated ribbed structure. The support unit 40 in the embodiment is L-shaped. The support unit 40 is mounted in the lower support slot 13 of the card base 10 by the lower convex portion 43, but it is not limited thereto. The lower convex portion 43 may be changed to a slot and the lower support slot 13 may be changed to a block according to the user.

The fragrance member 50 is a rectangular plate, and a first embedding hole 51 and at least one second embedding hole 52 penetrate through the fragrance member 50. The first embedding hole 51 corresponds in position and shape to the first embedding unit 14, and the second embedding hole 52 corresponds in position and shape to the second embedding unit 15. The fragrance member 50 is sleeved on the first embedding unit 14 by the first embedding hole 51 and sleeved on the second embedding unit 15 by the second embedding hole 52, so that the fragrance member 50 is fixed on the card base 10. In this embodiment, the fragrance member 50 is an aroma stone, but it is not limited thereto. The fragrance member 50 can be made of at least one material that can absorb fragrance oil, such as paper, bamboo, rattan, gypsum, ceramics, diatomaceous earth, wood, cloth, lava and crystal, combined with fragrance oil. The fragrance member 50 can continuously emit fragrance by replenishing the fragrance oil.

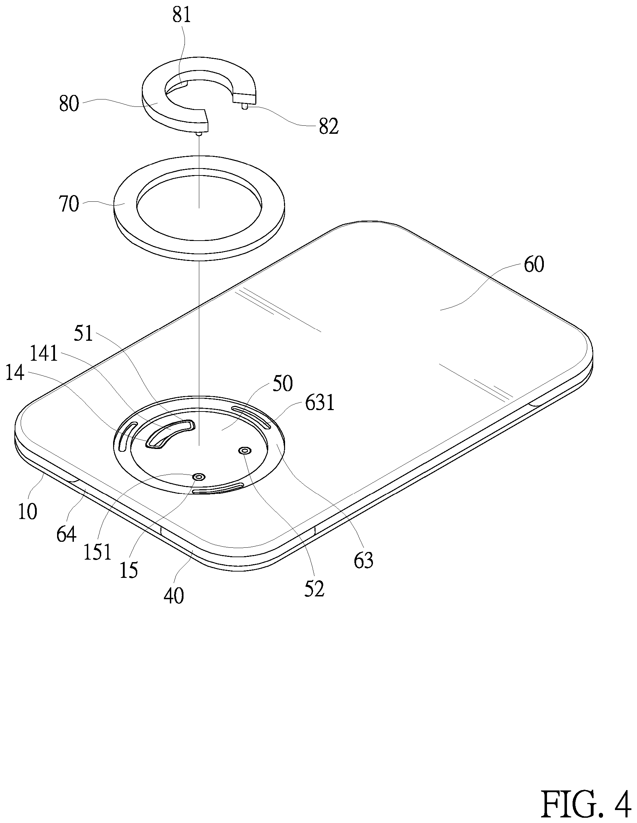

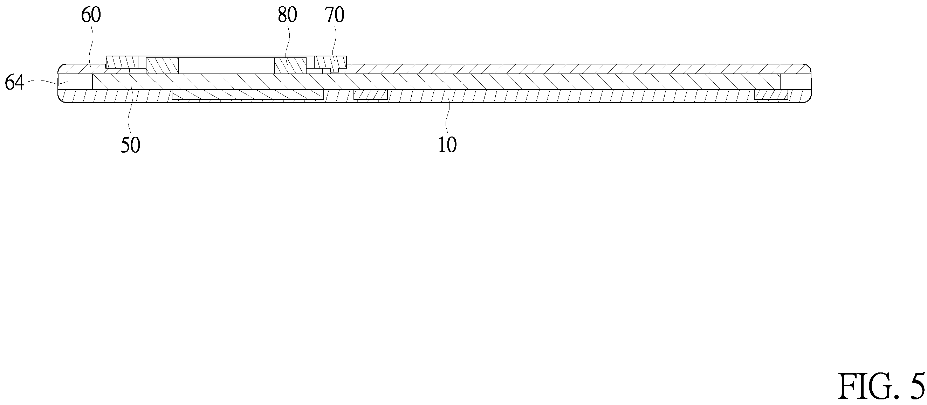

With reference to Fig. 2 to Fig. 5, a side surface of the outer cover 60 is concave to form multiple upper support slots 61, and each one of the upper support slots 61 corresponds in shape and position to each one of the lower support slots 13. Another side surface of the outer cover 60 is penetrated through by a cover hole 62, and the cover hole 62 corresponds in position to the first embedding hole 51 and the second embedding hole 52. The outer cover 60 is concave to form an annular slot 63 located on a periphery of the cover hole 62. The annular slot 63 is concave to form multiple embedding grooves 631, but it is not limited thereto, as the outer cover 60 may not form the cover hole 62 and the annular slot 63. The configuration of the outer cover 60 can be changed according to the user, as the support unit 40 may be a separate unit or may be integrally formed with the outer cover 60. The outer cover 60 is combined with the upper convex portion 42 of each one of the support units 40 by each one of the upper support slots 61, so that edges of the outer cover 60, each one of support units 40 and the card base 10 form multiple side penetrating holes 64, and each one of the side penetrating holes 64 is an elongated hole.

The decorative cover 70 is annular, and multiple embedding blocks 71 protrude from a side surface of the decorative cover 70. Each one of the embedding blocks 71 corresponds in shape to each one of the embedding grooves 631. The decorative cover 70 covers the cover hole 62 of the outer cover 60, and each one of the embedding blocks 71 is embedded in each one of the embedding grooves 631, but it is not limited thereto, as the magnetic fragrance card may not comprise the decorative cover 70.

The convex unit 80 is a C-shaped structure, and a first embedding convex portion 81 and at least one second embedding convex portion 82 protrude from a side surface of the convex unit 80. The first embedding convex portion 81 corresponds in shape to the first embedding slot 141, and the second embedding convex portion 82 corresponds in shape to the second embedding slot 151. The convex unit 80 is mounted through the decorative cover 70, and the cover hole 62 and the first embedding convex portion 81 are embedded in the first embedding slot 141, and the second embedding convex portion 82 is embedded in the second embedding slot 151, but it is not limited thereto, as quantities and shape of the first embedding convex portion 81 and the second embedding convex portion 82 may be changed according to the user.

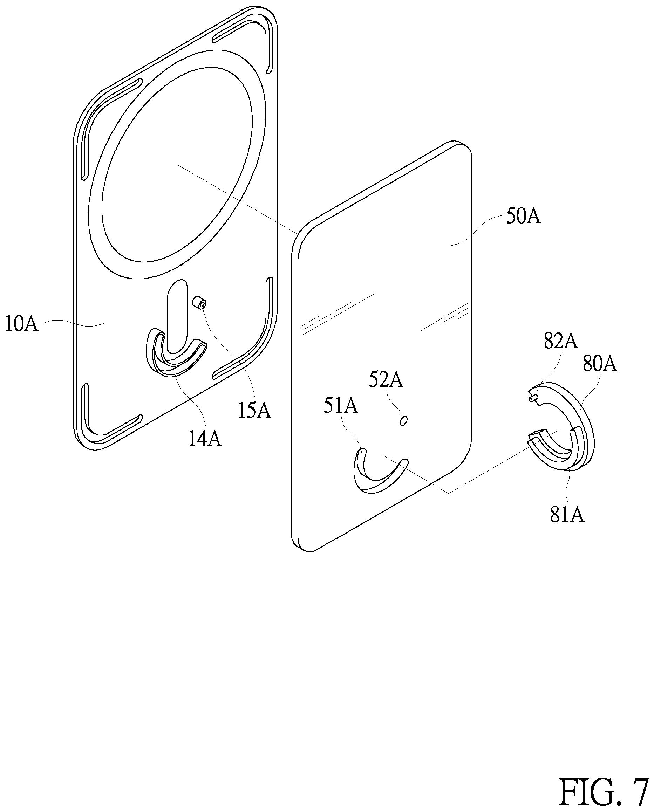

With reference to Fig. 7, in another embodiment, the first embedding unit 14A of the card base 10A is an arc-shaped block. Quantity of the second embedding unit 15A is one, and the shape and quantities of the first embedding hole 51A and the second embedding hole 52A of the fragrance member 50A and the shape and quantities of the first embedding convex portion 81A and the second embedding convex portion 82A of the convex unit 80A are changed corresponding to the second embedding unit 15A, so as to fix the convex unit 80A.

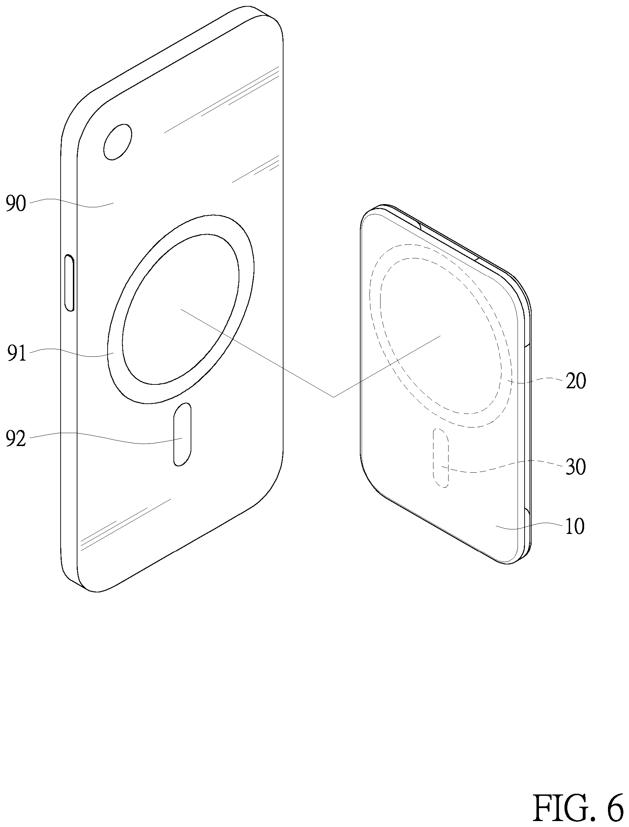

With reference to Fig. 2, Fig. 3, Fig. 5 and Fig. 6, the present invention is mounted on an electronic device 90 to supply fragrance. Precisely, the electronic device 90 is a mobile phone, but it is not limited thereto. A surface of the electronic device 90 comprises a magnetic area 91 and a magnetic positioning area 92. The card base 10 is magnetically mounted on the magnetic area 91 and the magnetic positioning area 92 of the electronic device 90 by the first magnetic member 20 and the second magnetic member 30, so that a back of the card base 10 and is connected to the surface of the electronic device 90, and the card base 10 and the electronic device 90 can be quickly combined or separated and is easy to carry.

In the aforementioned process, multiple side penetrating holes 64 are formed among and enclosed by the outer cover 60, the support units 40 and the card base 10. Precisely, each one of four sides of the magnetic fragrance card has a side penetrating hole 64 of a certain length, so that the fragrance member 50 disposed inside can have a larger volume, and also the fragrance of the fragrance member 50 can be emitted from each one of the side penetrating holes 64 and the cover hole 62 of the outer cover 60, so as to emit fragrance for a long time multi-directionally, enhancing the diffusion uniformity of the fragrance and the comfort of the user.

In the aforementioned process, the magnetic fragrance card comprises the convex unit 80, preventing the user or external objects from directly touching the fragrance member 50, thereby protecting the fragrance member 50 from being touched.

In the aforementioned process, since the first magnetic member 20, second magnetic member 30, and fragrance member 50 are all mounted on the card base 10, the overall thickness of the magnetic fragrance card can be reduced to be a slim design.

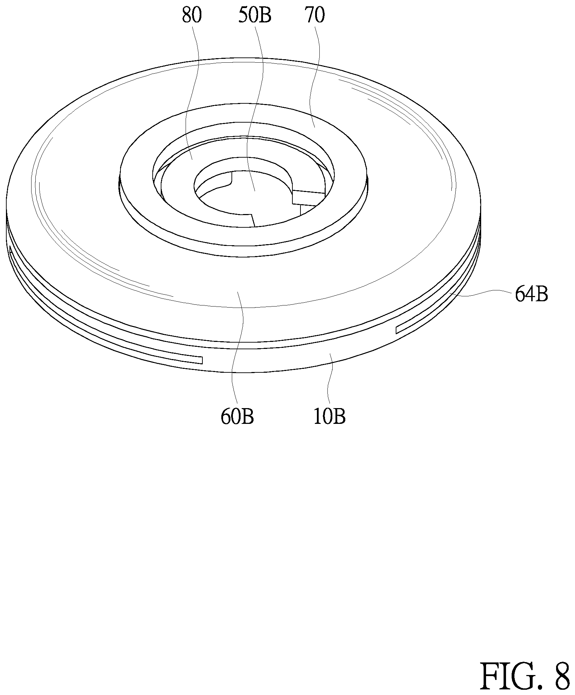

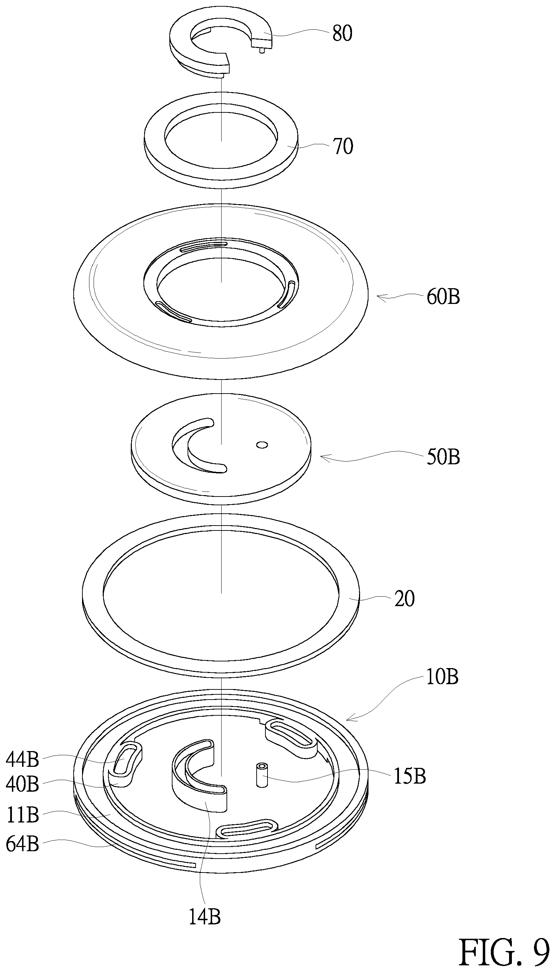

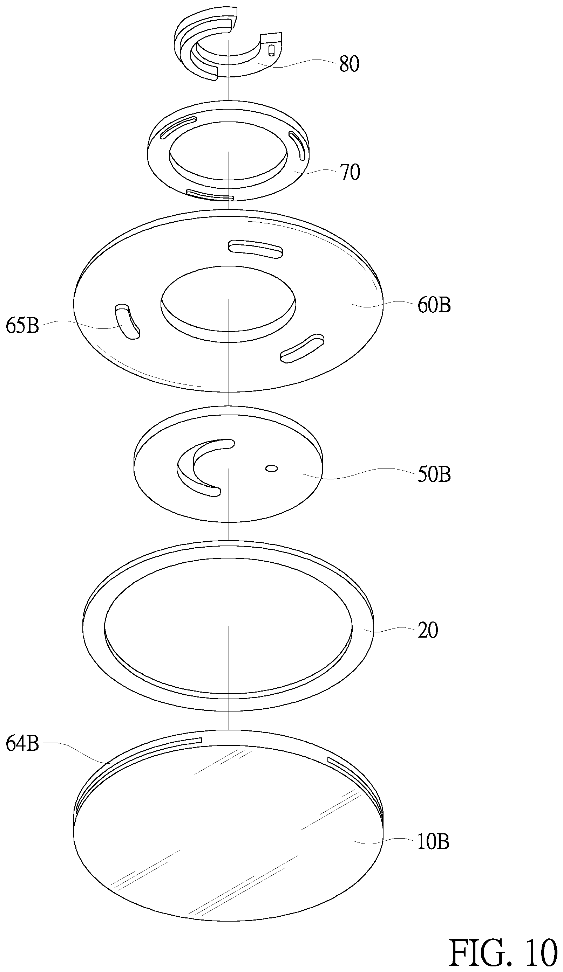

With reference to Fig. 8 to Fig. 10, the first magnetic member 20, the decorative cover 70, and the convex unit 80 in the second embodiment are the same as those in the first embodiment, and the main difference is that the card base 10B, the support unit 40B, the fragrance member 50B and the outer cover 60B are changed to a circular shape, and the magnetic fragrance card does not comprise the second magnetic member 30. Precisely, the card base 10B is a circular disc, and the first placement slot 11B is formed adjacent to a periphery of the card base 10. The card base 10 forms multiple side penetrating holes 64B located under the first placement slot 11B. The first placement slot 11B protrudes from an inside of each one of the support units 40B. The support unit 40B is an arc-shaped structure, and a top of each one of the support units 40B can be changed to an upper concave portion 44B. A first embedding unit 14B and a second embedding unit 15B are located among the support units 40B. The fragrance member 50B is a circular disc, and an outer diameter of the fragrance member 50B is smaller than an inner diameter formed by inner edges of each one of the support units 40B, but it is not limited thereto. The outer cover 60B is a circular disc, the upper support slots 61 on the side surface of the outer cover 60B are changed to the upper support blocks 65B, and the upper support blocks 65B correspond in shape and position to the upper concave portion 44B of the support units 40B, so that the outer cover 60B is combined with the support units 40B.

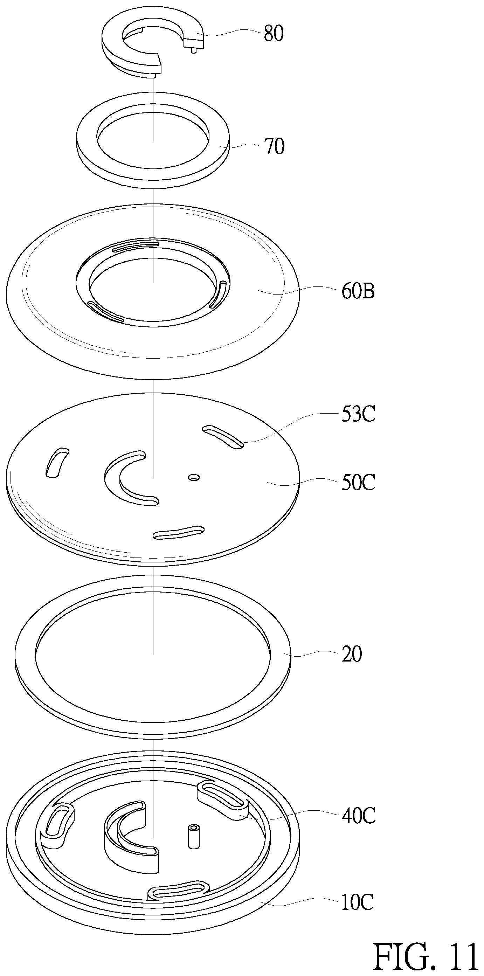

With reference to Fig. 11, in another embodiment, an outer diameter of the fragrance member 50C is equal to an outer diameter of the card base 10C. Multiple through-holes 53C penetrate through the fragrance member 50C. Each one of the through-holes 53C corresponds in shape and position to each one of the support units 40C, and the card base 10C does not form the side penetrating hole 64B of the second embodiment, but it is not limited thereto.

## Claims

1. A magnetic fragrance card **characterized in that** the magnetic fragrance card comprises:
a card base (10), a side surface of the card base (10) forming a first embedding unit (14), at least one second embedding unit (15), and a first placement slot (11); the first embedding unit (14) and the at least one second embedding unit (15) protruding from the card base (10), the first placement slot (11) concaved on the card base (10);
a first magnetic member (20) mounted in the first placement slot (11);
multiple support units (40) mounted on the side surface of the card base (10);
a fragrance member (50) penetrated through by a first embedding hole (51) and at least one second embedding hole (52), the first embedding hole (51) and the at least one second embedding hole (52) respectively corresponding in shape and position to the first embedding unit (14) and the at least one second embedding unit (15); the fragrance member (50) sleeved on the first embedding unit (14) by the first embedding hole (51), and the fragrance member (50) sleeved on the at least one second embedding unit (15) by the at least one second embedding hole (52);
an outer cover (60) penetrated through by a cover hole (62), the outer cover (60) covering the card base (10) and combined with each one of the support units (40);
a convex unit (80), a first embedding convex portion (81) and at least one second embedding convex portion (82) protruding from a side surface of the convex unit (80); the convex unit (80) mounted through the cover hole (62) and combined and fixed with the first embedding unit (14) by the first embedding convex portion (81), and the convex unit (80) combined and fixed with the second embedding unit (15) by the at least one second embedding convex portion (82).

2. The magnetic fragrance card as claimed in claim 1, wherein,
the side surface of the card base (10) adj acent to an outer edge of the card base (10) further forms multiple lower support slots (13);
each one of the support units (40) comprises a main body (41), an upper convex portion (42), and a lower convex portion (43); the main body (41) comprises an upper surface (411) and a lower surface (412), the upper convex portion (42) protrudes from the upper surface (411), and the lower convex portion (43) protrudes from the lower surface (412);
each one of the support units (40) mounted on a respective one of the lower support slots (13) by the lower convex portion (43);
a side surface of the outer cover (60) is concave to form multiple upper support slots (61), the outer cover (60) is combined and fixed with each one of the upper convex portions (42) by each one of the upper support slots (61); multiple side penetrating holes (64) formed between an edge of the outer cover (60), an edge of each one of the support units (40), and an edge of the card base (10).

3. The magnetic fragrance card as claimed in claim 2, wherein,
the card base (10) is a rectangular plate, and the card base (10) comprises two opposite side edges and two opposite end edges;
each one of the lower support slots (13) is an L-shaped groove and extends along one of the side edges and one of the end edges adjacent to the said side edge, each one of the support units (40) corresponds in shape to each one of the lower support slots (13).

4. The magnetic fragrance card as claimed in claim 1, wherein,
the first embedding unit (14) is an arc-shaped block, and a top surface of the first embedding unit (14) is concave to form a first embedding slot (141);
the at least one second embedding unit (15) is a columnar structure, and a top surface of the at least one second embedding unit (15) is concave to form a second embedding slot (151);
the first embedding convex portion (81) of the convex unit (80) is embedded in the first embedding slot (141), and the second embedding convex portion (82) is embedded in the second embedding slot (151).

5. The magnetic fragrance card as claimed in any one of claims 1 to 4, wherein,
the side surface of the card base (10) further forms a second placement slot (12), the second placement slot (12) is a straight groove, the first placement slot (11) is an annular groove, the first magnetic member (20) is an annular magnet and is mounted in the first placement slot (11), and a second magnetic member (30) is an elongated magnet and is mounted in the second placement slot (12).

6. The magnetic fragrance card as claimed in claim 1, wherein,
the card base (10) is a circular disc, the first placement slot (11) is formed adjacent to a periphery of the card base (10), each one of the support units (40) is an arc-shaped structure and protrudes from an inner side of the first placement slot (11), and the first embedding unit (14) and the at least one second embedding unit (15) are located among the support units (40);
the fragrance member (50) is a circular disc, the outer cover (60) is a circular disc, and a side surface of the outer cover (60) forms multiple upper support blocks, and each one of the upper support blocks is combined and fixed with each one of the support units (40).

7. The magnetic fragrance card as claimed in claim 6, wherein, an outer diameter of the fragrance member (50) is equal to an outer diameter of the card base (10).

8. The magnetic fragrance card as claimed in claim 6, wherein, the card base (10) forms multiple side penetrating holes (64) located under the first placement slot (11), and an outer diameter of the fragrance member (50) is smaller than an inner diameter formed by inner edges of each one of the support units (40).

9. The magnetic fragrance card as claimed in claim 6, wherein, an upper part of each one of the support units (40) is concave to form an upper concave portion, and each one of the upper support blocks is mounted through and fixed to the corresponding upper concave portion.

10. The magnetic fragrance card as claimed in claim 1, wherein, the outer cover (60) is concave to form an annular slot (63) located on a periphery of the cover hole (62), the annular slot (63) is concave to form multiple embedding grooves (631), multiple embedding blocks (71) protrude from a side surface of a decorative cover (70), and each one of the embedding blocks (71) is embedded in each one of the embedding grooves (631).
